# EUROPEAN PATENT APPLICATION

(11) **EP 3 446 651 A1**
(43) Date of publication of application: **27.02.2019**
(21) Application number: 18190642.1
(22) Date of filing: 24.08.2018
(51) Int. Cl.: A61B 17/80, A61B 17/15, A61B 17/17

(54) **ORTHOPEDIC PLATE, ORTHOPEDIC DEVICE AND METHOD OF ASSEMBLING AN ORTHOPEDIC PLATE**

(30) Priority: 24.08.2017 US 201715685689
(71) Applicant: Zimmer GmbH, 8404 Winterthur (CH)
(72) Inventor: HADDAD, Steven L., Glenview, IL 60025 (US); BOND, Paul, Chicago, IL 60642 (US)
(74) Representative: Mays, Julie

(57) **Abstract**

An orthopedic plate (514) comprising a frame portion (568) having a longitudinal body defining a longitudinal axis (510). The longitudinal body having at least one side arm (592A) extending from the longitudinal body transverse to the longitudinal axis. The frame portion also having a bearing (584) rotatably coupled with the at least one side arm, wherein the bearing defines an opening (570) configured to receive a fastener (574) for fastening the orthopedic plate to a body. The bearing includes an outer surface that is eccentric to the opening such that a position of the opening with respect to the frame portion is adjustable as the bearing rotates.

## Description

### CLAIM OF PRIORITY

This patent application is a continuation-in-part of U.S. Patent Application Serial No. 15/358,439, filed on November 22, 2016; which is a continuation of U.S. Patent Application Serial No. 13/767,462 filed on February 14, 2013 and issued as U.S. Patent No. 8,778,000 on July 15, 2014; which is a continuation of U.S. Patent Application Serial No. 13/708,213 filed on December 7, 2012 and issued as U.S. Patent No. 9,522,023 on December 20, 2016; which claims the benefit of priority under 35 U.S.C. §119(e) of U.S. Provisional Patent Application No. 61/569,052 filed December 9, 2011; each of which is hereby incorporated by reference herein in its entirety.

### TECHNICAL FIELD

The present disclosure relates generally to devices for and methods of repairing bones and/or bone joints and methods of assembling said devices. More specifically, the disclosure relates to an orthopedic plate or an orthopedic device for coupling bone segments, a method of doing the same, and a method of assembling an orthopedic plate.

### BACKGROUND

The statements in this section merely provide background information related to the present disclosure and may not constitute prior art.

When treating bone fractures, where a single bone is broken into two or more bone segments, a medical professional often desires to promote union between the two or more bone segments. The same is the case when a medical professional desires to cause or help to cause bone fusions, i.e., uniting two bones into one bone by eliminating a joint there between. When promoting union of two or more bone segments via standard biologic healing, whether the bone segments are pieces of a single bone or whether the bone segments are separate bones, it is often desirable to have precise alignment of bone segments and complete or substantially complete contact between the involved surfaces.

Alignment of the bone segments is desirable not only to enhance a union of bone segments, but also to prevent or reduce the likelihood of subsequent deformity following union. If malalignment is created at the time of fracture fixation, the ability of the bones to heal may be compromised and, if union is achieved, an alteration in force distribution may occur across formerly precisely balanced joints that may lead to increased contact stresses and subsequent arthritis. Joints often require precise balance to prevent portions of the cartilage from accelerated wear (wearing away the cartilage with repetitive cycles of loading), which may lead to early onset arthritis.

Thus, under the above-mentioned circumstances, the ability of the medical professional to achieve an outcome that both the patient and clinician approve of is often directly related to the quality of the reduction of the bone segments.

Traditionally, medical professionals, such as orthopedic surgeons, use plate fixation to hold the various bone segments into the correct position while they heal. The plates themselves are typically primarily alignment devices. While they may provide some element of structural support, if the fracture or fusion does not heal (nonunion), the plate and screw construct often eventually fails due to cyclic loading.

Dynamic compression plates have been used by medical professionals to attempt to promote biologic healing by creating a more complete and flush bond between bone segments. One type of dynamic compression plate includes oblong, rather than circular, holes to allow the medical professional to compress the fracture/fusion site by placing the screw against the side of the hole that is farthest from the fracture/fusion site. This type of compression plate is utilized with fasteners, such as screws, having a cone-shaped head with its largest diameter at the top of the fastener head. As the medical professional tightens the screw against the plate, the screw head engages the far end of the plate screw hole. Then, as the medical professional continues to tighten the fastener, the cone-shaped fastener head pushes the plate in a direction away from the fracture/fusion site as long as two conditions are met: (1) the bottom side of the plate is in contact with the bone to prevent the plate from moving downward as the fastener moves downward, and (2) the other end of the plate is secured to the bone on the opposite side of the fracture/fusion site.

The first of the above-mentioned conditions, namely that the bottom side of the plate is in contact with the bone while the fastener is being driven downward into the bone, may diminish the plate's effectiveness or render the plate unusable with bones that are not relatively flat. For example, as the medical professional tightens a fastener and causes the plate to contact an uneven bone surface, the bone may become distorted or otherwise damaged. Distortion of the fracture or fusion site may alter the alignment of said site or may limit the contact surface area between the bone segments. In either case, the desired goal of anatomic restoration of the bone or fusion site with maximal surface area available for healing may not be achieved. As a result, this type of dynamic compression plate may be undesirable for use with curved or uneven bone surfaces.

This type of dynamic compression plate may also be undesirable because the amount of compression is dependent on the screw height. In other words, the position of the plate along a first axis is dependent on the position of the fastener along a second axis that is generally perpendicular to the first axis. The dependent relationship between the plate and the screw height may not be desirable because it may prevent the medical professional from creating a desired compression force acting on the bone segments while the fasteners are at their desired positions.

Therefore, it is desirous to provide an orthopedic plate, device, or method that can be used with bone segments having various shapes while allowing dynamic compression of multiple bone segments and/or that can be used to create a desired compression force acting on the bone segments while the fasteners are at their desired positions.

### OVERVIEW

In overcoming the limitations and drawbacks of the prior art, the present orthopedic plate, device, and methods facilitate and/or provide dynamic compression between multiple bone segments.

In one aspect, an orthopedic plate is provided, comprising a frame portion, and a bearing rotatably coupled with the frame portion, wherein the bearing defines an opening configured to receive a fastener for fastening the orthopedic plate to a body, wherein the bearing includes an outer surface that is eccentric to the opening such that a position of the opening with respect to the frame portion is adjustable as the bearing rotates, and wherein the bearing includes at least a first ridge that is an anchoring ridge and a second ridge that is a locking ridge.

The anchoring ridge may have an inner diameter that is smaller than an inner diameter of the locking ridge. Furthermore, the anchoring ridge may be configured to mate with a first set of fastener threads and the locking ridge is configured to mate with a second set of fastener threads. The bearing may be configured to expand in diameter when the second set of fastener threads is received within the locking ridge.

The anchoring ridge may be configured to mate with a first set of fastener threads and the locking ridge is configured to receive a locking head. The bearing may be configured to expand in diameter when the locking head is received within the locking ridge.

The orthopedic plate may also include a second bearing rotatably coupled with the frame portion, wherein the second bearing defines a second opening configured to receive a second fastener for further fastening the orthopedic plate to a body, wherein the second bearing includes an outer surface that is eccentric to the second opening.

In another aspect an orthopedic plate is provided, having a frame portion and a bearing rotatably coupled with the frame portion, wherein the bearing defines an opening configured to receive a fastener for fastening the orthopedic plate to a body, wherein the bearing includes an outer surface that is eccentric to the opening such that a position of the opening with respect to the frame portion is adjustable as the bearing rotates and wherein the bearing includes at least one key hole to facilitate rotation of the bearing with respect to the frame portion.

The bearing may include at least two key holes to facilitate rotation of the bearing with respect to the frame portion. The bearing may also be configured to facilitate rotation of the bearing with respect to the frame portion while the fastener is received within the opening.

In one aspect, an orthopedic plate is provided, comprising a frame portion that can comprise a longitudinal body defining a longitudinal axis, wherein at least one side arm extends outward from the longitudinal body. A bearing can be rotatable coupled with the side arm, wherein the bearing defines an opening configured to receive a fastener for fastening the orthopedic plate to a body. The bearing is rotatable about a rotational axis, wherein the opening is offset from the rotational axis such that a position of the opening with respect to the side arm and the longitudinal axis is adjustable as the bearing rotates.

The longitudinal body can have two side arms each having a bearing with an offset opening, wherein a fastener can be inserted through each of the openings to engage one of two bone segments separated by a fracture. The longitudinal body can be laid across the two bone segments such that one of the two side arms is positioned against each bone segment on either side of the fracture. In this configuration, each of the side arms is operably connected to one of the two bone segments by a corresponding fastener, wherein the longitudinal body operably joins the two bone segments via the connection to each bone segment by the corresponding side arm and fastener.

The bearings can be rotated to draw the two bone segments together into engagement and close the gap defining the fracture. Each fastener can be inserted into the corresponding bone segment along an axis parallel to the ends of the bone segments defining the fracture such that rotation of the bearings can apply a compression force along the length of the fastener toward the fracture. In an example, the offset arrangement of the two side arms can cause greater compression forces at the edges of the contact surfaces between the two bone segments such as at the compact bone and/or periosteum of the bone. As certain bones or bone segments are hollow with the most rigid portion of the bone being the compact bone, the offset arrangement places the greatest compression force at those regions of the contact surfaces thereby improving the joining of the bones.

The side arms can be positioned to extend from opposite sides of the longitudinal body. The fastener of the first side arm engages the first bone segment on a first side of the longitudinal axis and the fastener of the second side arm engages the second bone segment on a second side of the longitudinal axis. In this configuration, rotating the bearings of the first and second bone segments moves the first and second bone segments into engagement along the fracture and applies compression pressure to the joined first and second bone segments from at least two opposing sides to the longitudinal axis, which more evenly joins the bone segments across the fracture.

The longitudinal body can be laid across the two bone segments and can comprise a planer body defining a plane. The side arms can extend from the longitudinal body along an axis transverse to the plane of the longitudinal body. In an embodiment, the side arms can be angled relative to the longitudinal body along mirrored or different axes to conform to the contour of the first or second bone segment. The fastener of the first side arm is inserted into the first bone segment along a first axis and the fastener of the second side arm is inserted into the second bone segment along a second axis transverse to the first axis. The fasteners can each extend through the first and second bone segments such that the fasteners apply compression force toward the fracture across the entire fracture along first and second axis. The transverse angle of the first axis to the second axis more evenly applies compression pressure across the face of the fracture.

In yet another aspect, an orthopedic device is provided, configured to facilitate cutting at least one of first and second bone segments of a body and comprising a jig configured to be secured to the first and second bone segments of the body and a cutting guide coupled with the jig, wherein a position of the cutting guide with respect to the jig is adjustable along a first axis.

In another aspect, an orthopedic device is configured to facilitate coupling first and second bone segments of a body and comprising a jig having a first arm configured to be secured to the first bone segment, a second arm configured to be secured to the second bone segment, and a jig adjustment mechanism configured to adjust the position of the first arm with respect to the second arm to adjust a distance between the first and second bone segments.

In yet another aspect, a method of coupling first and second bone segments of a body is provided, comprising coupling a first arm of a jig with the first bone segment and coupling a second arm of the jig with the second bone segment, coupling a cutting guide with the jig to facilitate cutting at least one of the first and second bone segments, cutting at least one of the first and second bone segments, decoupling the cutting guide from the jig, coupling a plate holding mechanism with the jig, coupling an orthopedic plate with the plate holding mechanism, adjusting the plate holding mechanism so as to move the orthopedic plate into a desired position with respect to the first and second bone segments, securing a first portion of the orthopedic plate to the first bone segment and securing a second portion of the orthopedic plate to the second bone segment, adjusting at least one of the following: a position of at least one of the first and second arms of the jig so as to adjust a distance between the first and second bone segments and a plate adjusting mechanism to adjust a distance between the first and second bone segments.

Further objects, features and advantages of the orthopedic plate, device, and method will become readily apparent to persons skilled in the art after a review of the following description, with reference to the drawings and claims that are appended to and form a part of this specification.

This overview is intended to provide an overview of subject matter of the present patent application. It is not intended to provide an exclusive or exhaustive explanation of the present subject matter. The detailed description is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Figure 1 shows an isometric view of an orthopedic device embodying principles of the present disclosure and having a jig with first and second arms and an orthopedic plate;
Figure 2 is a top view of the orthopedic device shown in Figure 1;
Figure 3 is a front view of the orthopedic device shown in Figure 1;
Figure 4 is a side view of the orthopedic device shown in Figure 1;
Figure 5a is a top view of the orthopedic plate shown in Figure 1 where first and second bearings of the orthopedic plate are each in a non-compressed position;
Figure 5b is a top view of the orthopedic plate shown in Figure 1 where the first and second bearings of the orthopedic plate are each in a compressed position;
Figure 6 is a cross-sectional view of the orthopedic plate shown in Figure 5b taken along line 6-6;
Figure 7 is an isometric view of an alternative embodiment of an orthopedic device coupled with first and second bone segments of a patient's body;
Figure 8 shows the orthopedic device shown in Figure 7, further including a cutting guide coupled with the jig;
Figure 9 shows the orthopedic device shown in Figure 7, further including a surgical saw received within a slot of the cutting guide;
Figure 10 shows the orthopedic device shown in Figure 7, where portions of the first and second bone segments have been removed to form complimentary bonding surfaces;
Figure 11 shows the orthopedic device shown in Figure 7, where a medical professional is adjusting the position of the jig first arm with respect to the jig second arm, thereby adjusting the position of the first bone segment with respect to the second bone segment;
Figure 12 shows the orthopedic device shown in Figure 7, where the medical professional has adjusted the position of the jig first arm with respect to the jig second arm such that the first and second bone segments are abutting each other;
Figure 13 shows the orthopedic device shown in Figure 7 coupled with a plate holding mechanism, where the plate holding mechanism is configured to position an orthopedic plate with respect to the first and second bone segments;
Figure 14 shows the orthopedic device shown in Figure 7, further including drill guides coupled with the orthopedic plate and where the medical professional is drilling along the drill guides and into the first and second bone segments;
Figure 15 shows the orthopedic device shown in Figure 7, where a medical professional is securing the orthopedic plate to the first and second bone segments;
Figure 16 shows the orthopedic plate shown in Figure 15, where the jig and orthopedic plate holding mechanism have been decoupled from the orthopedic plate;
Figure 17 shows an isometric view of the orthopedic plate shown in Figure 15 and a plate adjustment mechanism configured to mate with the orthopedic plate and adjust the position of bearings therein;
Figure 18A shows an isometric view of the orthopedic plate and plate adjustment mechanism shown in Figure 17, where the orthopedic plate and plate adjustment mechanism are mated with each other;
Figure 18B shows an isometric view of an orthopedic plate and a plate adjustment mechanism configured to mate with the orthopedic plate and adjust the position of bearings therein, the plate adjustment mechanism having a fastener port for turning the fastener with the plate adjustment mechanism coupled to the orthopedic plate;
Figure 19 shows an isometric view of the underside of the bearing for the orthopedic plate shown in Figure 17;
Figure 20 shows the bearing being coupled with the orthopedic plate frame;
Figure 21 shows the bearing coupled with the orthopedic plate frame;
Figure 22 shows a fastener configured to couple an orthopedic plate to one or more bone segments, wherein the fastener includes first and second threads;
Figure 23 shows an alternative embodiment of an orthopedic plate embodying principles of the present disclosure;
Figure 24 is another alternative embodiment of an orthopedic plate embodying principles of the present disclosure;
Figure 25 is a fastener coupled with the orthopedic plate shown in Figure 24;
Figure 26 is a cross-sectional view of the orthopedic plate shown in Figure 24 taken along line 26-26;
Figure 27 is an alternative embodiment of a fastener configured to couple an orthopedic plate to one or more bone segments;
Figure 28 is an isometric view of an orthopedic plate embodying principles of the present disclosure;
Figure 29A is a top view of the orthopedic plate shown in Figure 28 prior to adjustment of the orthopedic plate to draw a first bone segment and a second bone segment together;
Figure 29B is a top view of the orthopedic plate shown in Figure 28 after adjustment of the orthopedic plate to draw the first bone segment and the second bone segment together;
Figure 30 is a longitudinal view of the orthopedic plate shown in Figure 28;
Figure 31A is a side view of the orthopedic plate shown in Figure 28 prior to adjustment of the orthopedic plate to draw a first bone segment and a second bone segment together;
Figure 31B is a side view of the orthopedic plate shown in Figure 28 after adjustment of the orthopedic plate to draw the first bone segment and the second bone segment together.

### DETAILED DESCRIPTION

The following description is merely exemplary in nature and is not intended to limit the present disclosure, application, or uses.

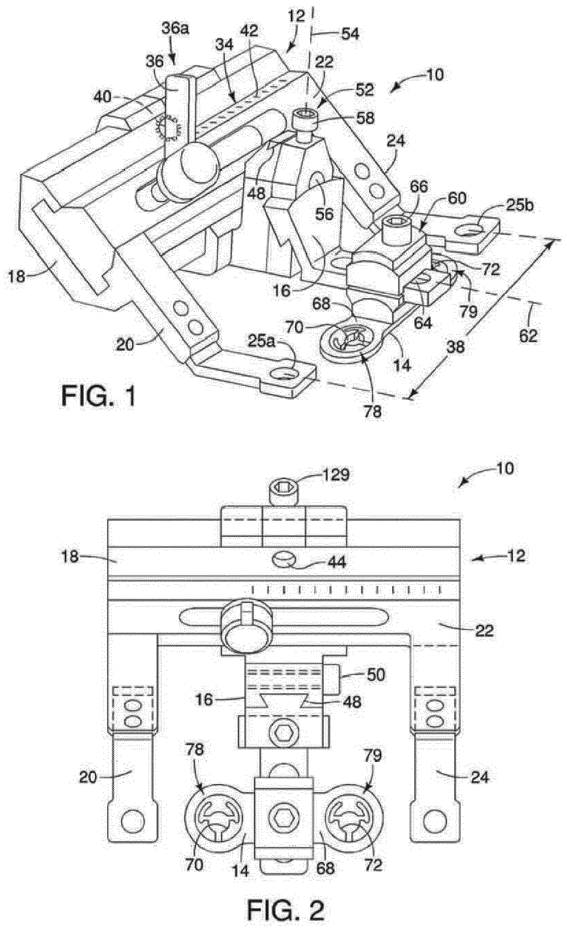

Referring now to the drawings, Figure 1 shows an orthopedic device 10 for coupling bone segments of a patient's body. The orthopedic device 10 includes a jig 12, an orthopedic plate 14, and a plate holding mechanism 16. Although these components are shown being used with each other, many or all of the components may be used independently of each other. For example, a medical professional may choose to use the orthopedic plate 14 without using the jig 12 or may choose to use the jig 12 with another orthopedic plate or another device altogether.

The jig 12 shown in the figures is coupled with the patient's body to facilitate installation of the orthopedic plate 14. The jig 12 shown in the figures includes a first portion 18 having a first arm 20 that is able to be coupled with the patient's body and a second portion 22 having a second arm 24 that is also able to be coupled with the patient's body.

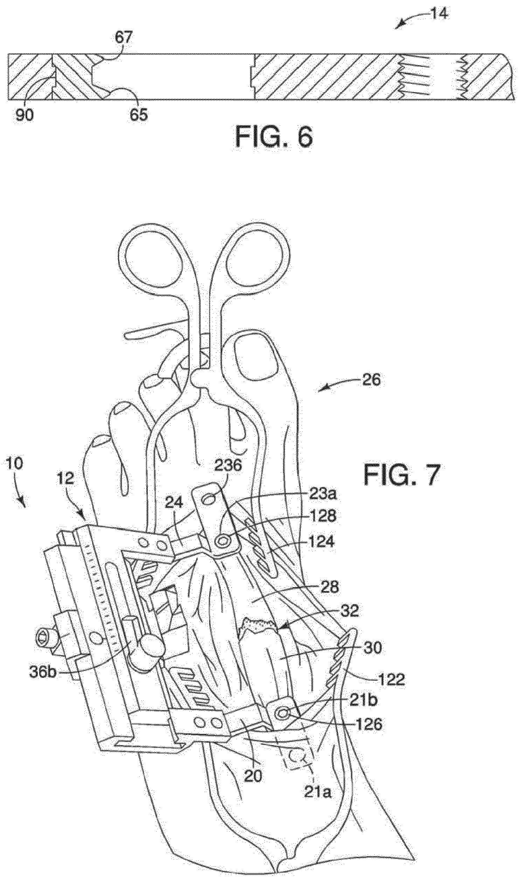

For example, Figure 7 shows a patient's body 26, particularly a patient's foot, having a first bone segment 28 and a second bone segment 30 that have been separated via a fracture 32. In the embodiment of the jig 12 shown in Figure 7, the first and second arms 20, 24 each have two fastener holes 21a, 21b, 23a, 23b that may be used to secure the jig 12 to the bone segments, whereas the first and second arms 20, 24 of the jig 12 shown in Figure 1 each include one fastener hole 25a, 25b. Although the figures focus on the foot portion of the patient's body 26, the present disclosure may be used with any suitable portion of a patient's body, such as the hands, ankles, wrists, legs, arms, oral or maxillofacial areas, or any other portion of a patient's body. The patient shown in the drawings is human, but the devices and methods disclosed herein may also be used on animals, such as through veterinarian medicine, and thus the term "medical professional" includes all types of medicine, including veterinarian medicine.

Referring to Figure 7-16, although these figures show first and second bone segments 28, 30 of a metatarsal bone that have been separated via a fracture 32, the present disclosure may be used with any suitable injury, condition, disease, malady, or weakness to a patient's body, such as a fracture, fusion, crack, or damaged joint. The term "bone segments" may refer to two portions of a single bone or two different bones. The metatarsal bone shown in the figures is, for illustrative purposes, longer and extends more proximal than a typical metatarsal bone in a normal adult patient. Also, for illustrative purposes, other bones of the patient's foot are not shown. If the jig 12 was used for a fusion application, rather than a fracture, in a similar area of a patient's foot, then the first and second arms of the jig likely would be secured to a metatarsal distally and a cuneiform proximally. However, as discussed above, the orthopedic device 10 may be used with any suitable bone segments.

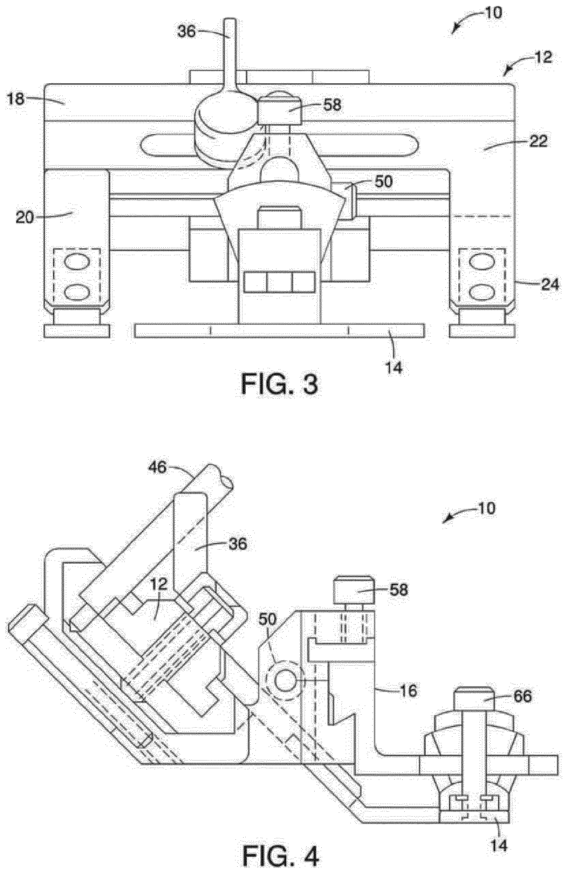

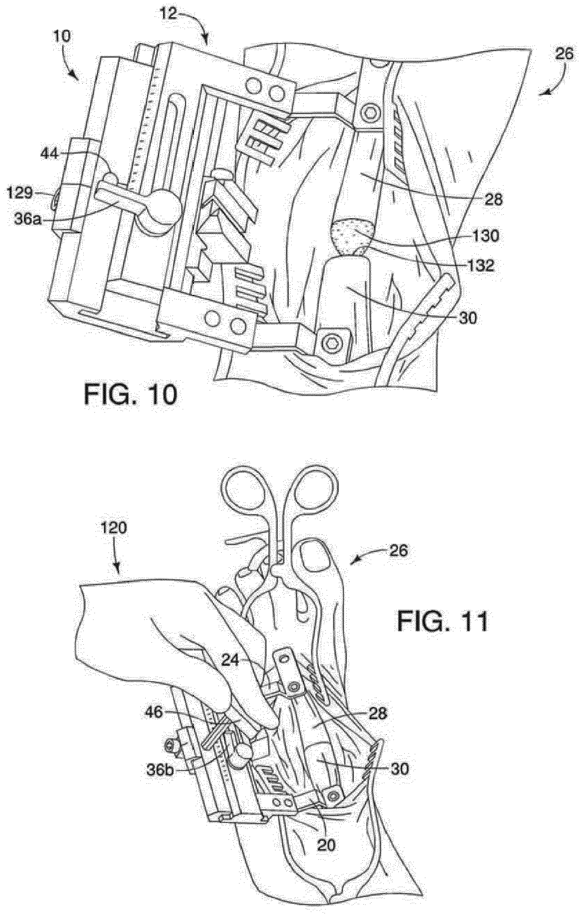

Referring to Figures 1-4, the second portion 22 of the jig 12 is slidably received within the first portion 18 so that the respective portions 18, 22 are movable with respect to each other. The first and second portions 18, 22 shown in the figures further include a jig adjustment mechanism 34 and a locking key 36 for adjusting a distance 38 between the first arm 20 and the second arm 24 of the jig 12. The locking key includes a locked position 36a (Figures 1-4, 8-10, 12-15), in which the first and second portions 18, 22 of the jig 12 are movable with respect to each other, and an unlocked position 36b (Figures 7 and 11), in which the first and second portions 18, 22 of the jig 12 are not movable with respect to each other. The jig 12 shown in the figures also includes a gear 40 and track 42, such as a rack and pinion, that cooperate to define the jig adjustment mechanism 34, but any other suitable adjustment mechanism may be used. The gear 40 in the figures is located within a cavity defined by the jig first portion 18 and is accessible from outside the jig 12 via a key hole 44 in the jig 12. The orthopedic device 10 further includes an adjustment key 46 (Figures 4, 11) configured to be inserted into the key hole 44 (Figures 2, 8- 10) and rotate the gear 40, thereby adjusting the distance 38 between the first and second arms 20, 24. When the jig 12 is coupled with the first and second bone segments 28, 30, by turning the adjustment key 46, a medical professional is able to adjust the relative position of the first and second bone segments 28, 30 with respect to each other. As a result, a medical professional is able to use the jig 12 to cause the first and second bone segments to dynamically compress, or cause the bone segments 28, 30 to come into contact with each other, and potentially promote biological healing.

As best shown in Figures 1 and 2, the plate holding mechanism 16 is coupled to the jig 12 via a tab-slot connection 48 and securing pin 50 (Figure 2) which secures the tab-slot connection 48. The plate holding mechanism 16 further includes a vertical adjustment mechanism 52 configured to adjust the position of the orthopedic plate 14 along a y-axis 54 (Figure 1). For example, the vertical adjustment mechanism 52 shown in the figures includes a tab-slot connection 56 and securing pin 58 securing the tab-slot connection 56 (Figure 1). The plate holding mechanism 16 further includes a horizontal adjustment mechanism 60 configured to adjust the position of the orthopedic plate 14 along an x-axis 62 (Figure 1). For example, the horizontal adjustment mechanism 60 shown in the figures includes a tab-slot connection 64 and securing pin 66 securing the tab-slot connection 64 (Figure 1). By using the vertical adjustment mechanism 52 and the horizontal adjustment mechanism 60, a medical professional is able to adjust the position of the orthopedic plate 14 with respect to the first and second bone segments 28, 30 so as to properly align the orthopedic plate 14 before coupling it with the bone segments 28, 30. In an alternative embodiment, the vertical adjustment mechanism and the horizontal adjustment mechanism include gear and track mechanisms such as the rack and pinion mechanism discussed with respect to the jig adjustment mechanism 34.

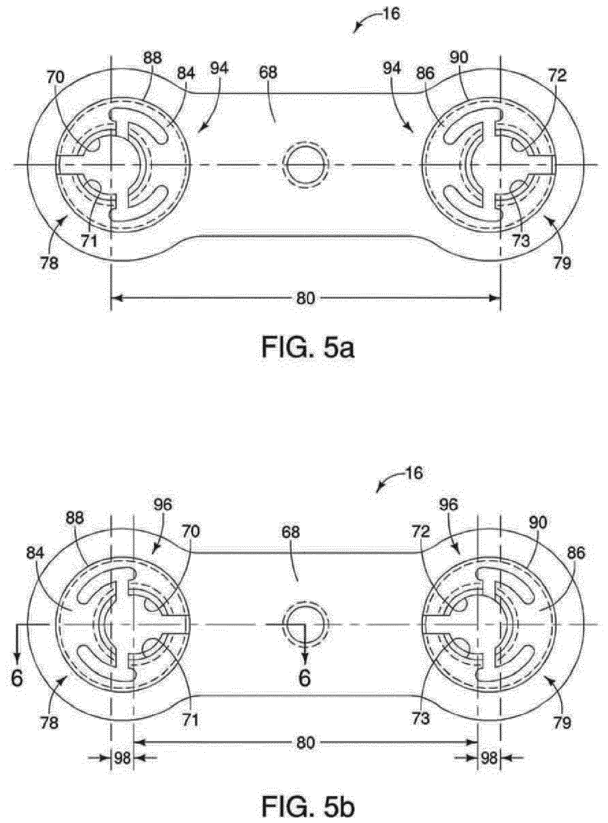

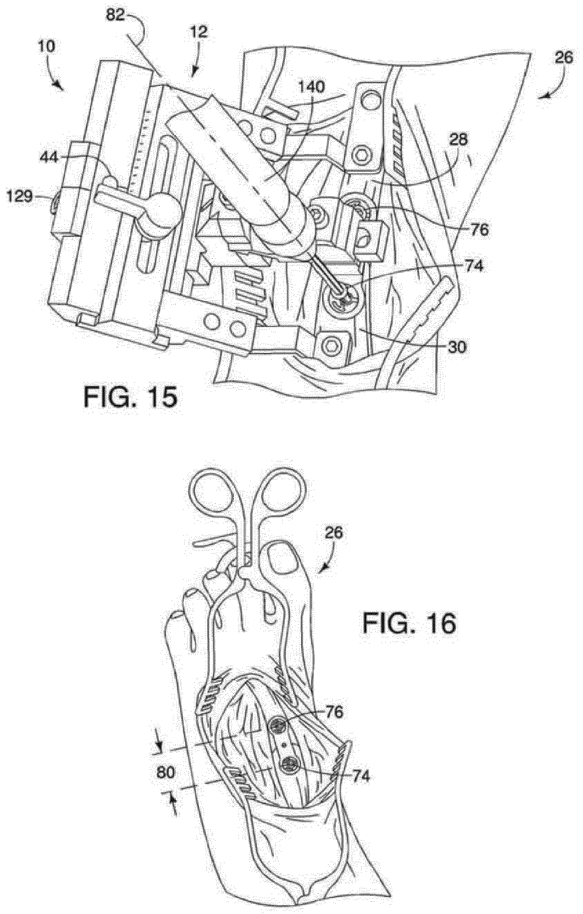

As is best shown in Figures 5a and 5b, the orthopedic plate 14 includes a frame portion 68 and first and second plate adjustment mechanisms 78, 79 that are each configured to adjust a distance 80 (Figure 16) between first and second fasteners 74, 76 (Figures 15, 16), respectively. The plate adjustment mechanisms 78, 79 shown in the figures include a first bearing 84 that is rotatable with respect to the frame portion 68 and a second bearing 86 that is rotatable with respect to the frame portion 68.

The orthopedic plate 14 shown in the figures defines a first opening 70 and a second opening 72 that are configured to receive the first and second fasteners 74, 76, to couple the orthopedic plate 14 to the first and second bone segments 28, 30. The distance 80 is measured at the center of each of the fasteners 74, 76 and is therefore, in the embodiments shown in the figures, the same distance as that measured from the respective centers of each of the openings 70, 72 (Figures 5a, 5b).

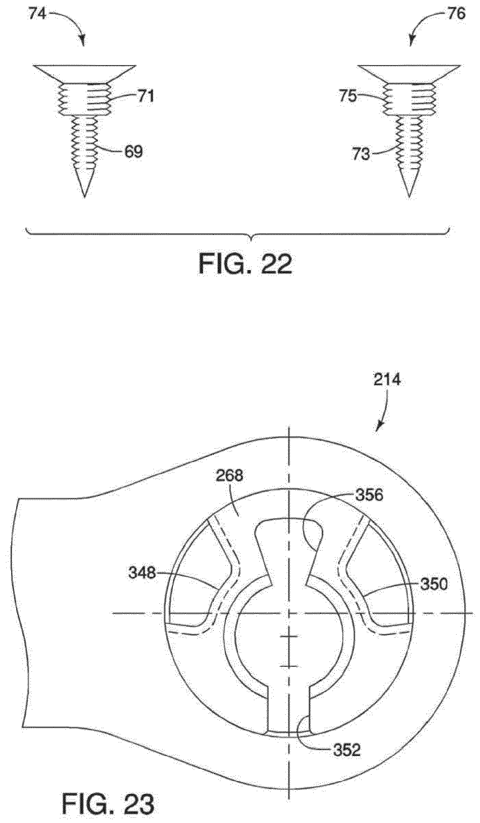

As best shown in Figures 5a, 5b, and 6, the first and second bearings 84, 86 each include two annular ridges: a anchoring ridge 65 and a locking ridge 67. The anchoring ridge 65 defines the opening 70, 72 in each of the bearings 84, 86. As best shown in Figure 22, the fasteners 74, 76 each include two sets of threads: anchoring threads 69, 73 and locking threads 71, 75. The anchoring threads 69, 73 are configured to mate with the anchoring ridges 65 in the first and second bearings 84, 86, respectively, while the fasteners 74, 76 are being screwed into the bone segments 28, 30. The locking threads 71, 75 are configured to mate with the locking ridge 67 when the fasteners 74, 76 are substantially or completely screwed down into the bone segments 28, 30 so as to prevent the bearings from rotating with respect to the plate portion 68. More specifically, the diameter of the locking threads 71, 75 is sized so as to cause the bearings 84, 86 to expand and form a friction engagement with the plate portion 68. The bearings 84, 86 are able to rotate with respect to the plate portion 68 except when the locking threads 71, 75 cause the bearings 84, 86 to expand and form a friction engagement with the plate portion 68. The locking feature of the fasteners 74, 76 make ti easier and more effective for the medical professional to "float" the orthopedic plate 14 above the bone segments 28, 30 (i.e., to space the plate 14 apart from the bone segments 28, 30). The locking feature of the fasteners 74, 76 also may improve or stabilize the connection between the bone segments 28, 30, even when a jig 12 is not being used. The locking feature also prevents or minimizes undesired rotation the bearings 84, 86 after the plate 14 has been installed.

Referring to Figures 5a and 5b, the bearings 84, 86 each include an outer surface 88, 90 that is eccentric to the inner surface (e.g., the anchoring ridge 65) of the bearing 84, 86. In other words, the outer surface 88, 90 of the bearings 84, 86 and the anchoring ridge 65 each generally define circles that have different centerpoints. As a result, the distance 80 is adjusted as one of the bearings 84, 86 is rotated with respect to the frame portion 68. A medical professional is able to use the orthopedic plate 14 to cause the first and second bone segments 28, 30 to dynamically compress, or cause the bone segments 28, 30 to come into contact with each other, and potentially promote biological healing.

The plate adjustment mechanism 78 shown in the figures is configured to be able to adjust the distance 80 while the orthopedic plate 14 is spaced apart from at least one of the first and second bone segments 28, 30, as is measured generally along a fastener axis 82 (Figure 15). In other words, the orthopedic plate 14 does not need to abut the bone segments 28, 30 to be able to adjust the distance 80, thereby permitting dynamic compression of the bone segments 28, 30 while minimizing, reducing, or avoiding distortion to the bone segments 28, 30 by the orthopedic plate 14. Utilizing the orthopedic plate 14 in this manner may be particularly advantageous where the bone segments 28, 30 are uneven along the length of the orthopedic plate 14. However, the orthopedic plate 14 is also usable and adjustable when it is abutting the bone segments 28, 30. In some cases, such as where the bone segments 28, 30 are relatively flat, it may be desirable for the orthopedic plate 14 to abut the bone segments 28, 30.

As is illustrated in Figures 5a and 5b, the distance 80 is largest (and the orthopedic plate 14 offers the least amount of compression) when the first and second bearings 84, 86 are each rotated such as to be in a non-compressed position 94 (i.e., where the centerpoints of the first and second bearings 84, 86 are furthest from each other). Conversely, the distance 80 is smallest (and the orthopedic plate 14 offers the maximum amount of compression) when the first and second bearings 84, 86 are each rotated such as to be in a compressed position 96 (i.e., where the centerpoints of the first and second bearings 84, 86 are closest to each other). Each of the first and second bearings 84, 86 defines a compression adjustment distance 98. The compression adjustment distance 98 is the distance measured along the longitudinal axis of the orthopedic plate 14 between the centerpoint of a bearing in the non-compressed position 94 and the compressed position 96. The distance 80 is therefore adjustable by an amount equal to the compression adjustment distance 98 of the first bearing 84 plus the compression adjustment distance 98 of the second bearing 86. In the embodiment shown in Figures 5a and 5b, the orthopedic plate 14 has a compression adjustment distance of approximately 1.5 millimeters, thereby allowing a medical professional to adjust the distance 80 of the orthopedic plate 14 by approximately 3.0 millimeters.

Another advantage to the orthopedic plate 14 shown in Figures 5a and 5b is that it allows a medical professional to adjust a horizontal distance (i.e., the distance 80) of the first and second fasteners 74, 76 independently of a vertical positioning (i.e., the position of the fasteners 74, 76 along the fastener axis 83) of the first and second fasteners 74, 76. This configuration allows a medical professional to have more control over the position (both horizontally and vertically) of the orthopedic plate 14 when coupling the same with the first and second bone segments 28, 30.

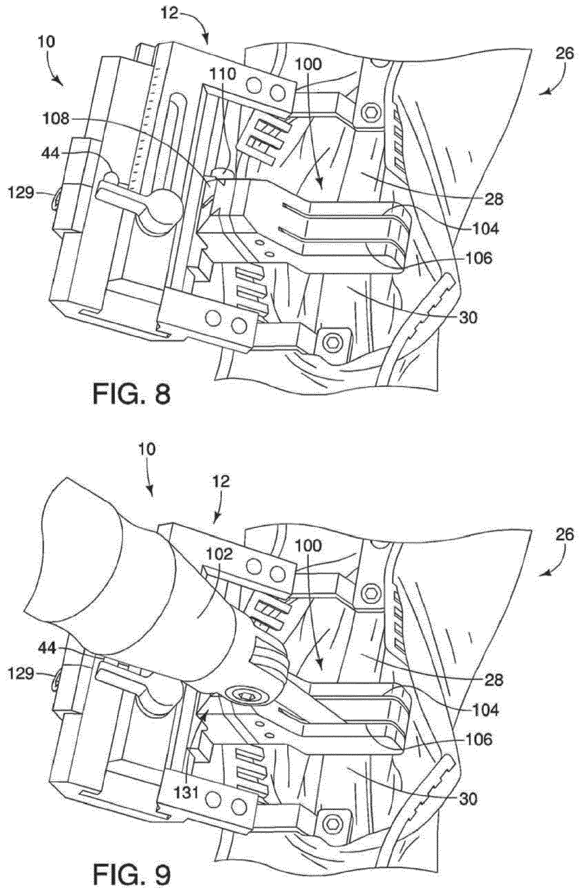

The orthopedic device 10 shown in the figures also includes a cutting guide 100 coupled with the jig 12 and configured to guide a surgical saw 102 or other cutting instrument. For example, the cutting guide 100 has a pair of cutting slots 104, 106 configured to receive the surgical saw 102 and allow a medical professional to cut through the bone segments 28, 30 in a relatively straight line by following the slots 104, 106. Often times, a medical professional will desire or need to cut opposing faces of bone segments 28, 30 so as to create two complimentary surfaces that will easily and effectively achieve a union through normal biological healing. It is often advantageous for the complimentary surfaces to be flat surfaces that are generally perpendicular to the longitudinal axis of the bone(s). The cutting guide is adjustable along the y-axis 54 (Fig. 1) by way of a tab-slot connection 108 and a securing pin 110 (Fig. 8). The cutting guide is adjustable along the x-axis 62 (Fig. 1) by way of the jig adjustment mechanism 34 (Fig. 1).

For illustrative purposes, a method of coupling first and second bone segments 28, 30 of a patient's body 26 is herein described. A medical professional (generally designated by numeral 120 in Figure 11) makes an incision in the patient's body 26 and exposes the bone segments 28, 30 to be coupled via clamps 122, 124. As shown in Figure 7, the medical professional couples the first arm 20 of the jig 12 with the first bone segment 28 and couples the second arm 24 of the jig 12 with the second bone segment 30 by using fasteners 126, 128. Turning to Figures 8 and 9, the medical professional then couples the cutting guide 100 with the jig 12 to facilitate cutting at least one of the first and second bone segments 28, 30 with the surgical saw 102. For example, the cutting guide 100 may be coupled with the jig 12 via a setscrew 129 (Figs. 2, 9) and a dovetailed holding clamp 131 (Fig. 9). The cutting slots 104, 106 shown in the figures are approximately 3.0 millimeters apart from each other, but may have any suitable distance therebetween. Also, the medical professional can adjust the distance between the two cuts by making a first cut in the first bone segment 28 and then horizontally adjusting the position of the cutting guide 100 via the securing pin 110. The medical professional then decouples the cutting guide 100 from the jig 12.

As shown in Figure 10, after cutting the bone segments 28, 30 preferably have flat, complimentary surfaces 130, 132 that will promote union when said surfaces are compressed together. As shown in Figure 11, with the locking key 36 in the unlocked position 36b, the medical professional 120 rotates the adjustment key 46 so as to move the first and second arms 20, 24 with respect to each other. For example, the medical professional 120 rotates the adjustment key 46 until the bone segments 28, 30 are in contact with each other or close to being in contact with each other. The medical professional may also, or alternatively, adjust the distance between the bone segments 28, 30 by unlocking the locking key 36 and manually pressing together or pulling apart the first and second portions of the jig 12.

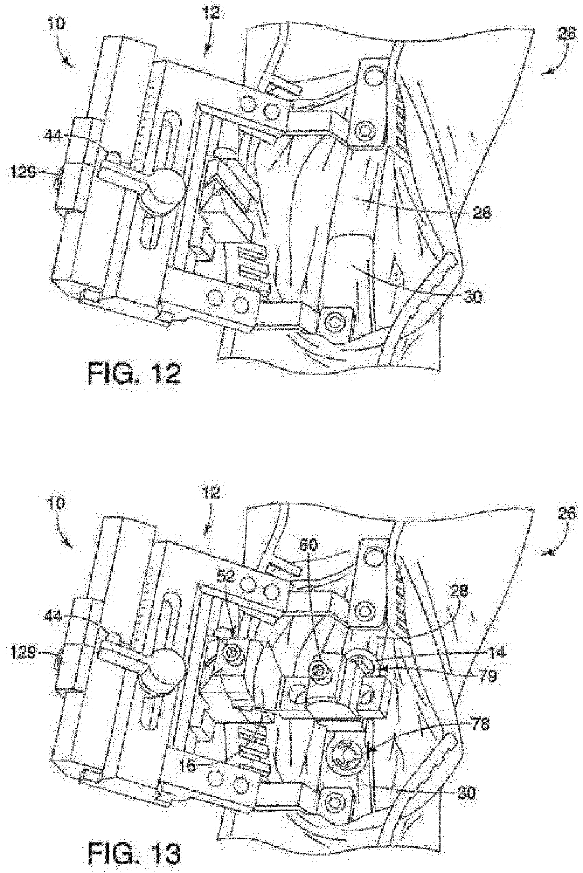

Once the arms 20, 24 of the jig 12 are positioned as desired, the medical professional then moves the locking key 36 into the locked position 36a (Figure 12) thereby coupling the plate holding mechanism 16 with the jig 12. The medical professional can adjust the horizontal or vertical position of the orthopedic plate 14 via the vertical adjustment mechanism 52 and the horizontal adjustment mechanism 60, respectively.

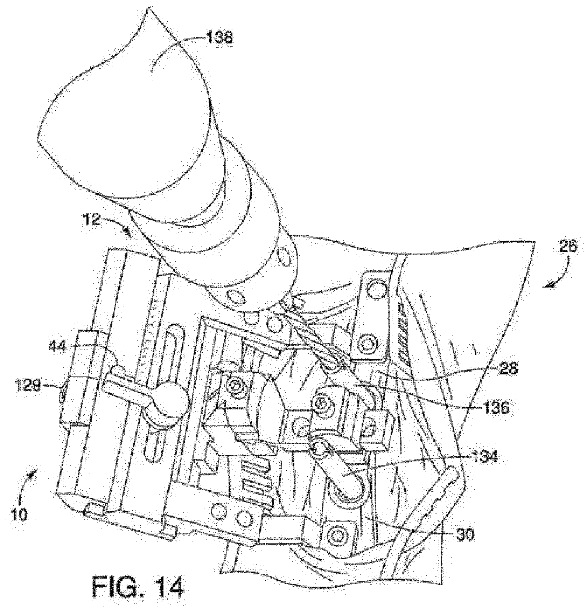

As shown in Figure 14, once the orthopedic plate 14 is in the desired position with respect to the bone segments 28, 30, the medical professional secures drilling guides 134, 136 to the orthopedic plate 14 and uses a surgical drill 138 to drill through the drill guides 134, 136 and into the bone segments 28, 30. The drilling guides 134, 136 shown in the figures are perpendicular to the orthopedic plate 14 and are coupled therewith by a threaded connection. Specifically, the drilling guides 134, 136 mate with the locking ridge 67. The inside diameter of the drilling guides 134, 136 corresponds to the diameter of the first and second openings 70, 72 (i.e., the diameter defined by the anchoring ridge) to assist with alignment of the drill bit as it creates holes in the bone segments 28, 30. The medical professional then removes the drilling guides 134, 136 from the orthopedic plate 14 and, as is shown in Figure 15, secures a first portion of the orthopedic plate 14 to the first bone segment 28 and secures a second portion of the orthopedic plate 14 to the second bone segment 30. For example, the medical professional shown in Figure 15 is securing the orthopedic plate 14 to the bone segments 28, 30 via the fasteners 74, 76 and a screwdriver 140.

When the fastener heads are flush with the bearings, the bearings outwardly expand, thereby locking the bearings in place with respect to the orthopedic plate frame portion and prevent rotation of the bearing. When the bearing is expanded (and thus locked) it forms an interference fit with the orthopedic plate frame portion, thereby substantially or completely preventing the bearing from back spinning into an uncompressed position under physiologic loads. The bearings 84, 86 include bearing key holes 148, 150 that facilitate rotation of the bearings 84, 86, as well as facilitate compression of the bone segments 28, 30, as will be described in more detail below. In other words, the first and second bearings 84, 86 are configured to facilitate rotation of the bearings with respect to the frame portion 68 while the fasteners 74, 76 are received within the first and second openings 70, 72, respectively.

Next, the plate holding mechanism 16 is decoupled from the orthopedic plate 14 and the jig is decoupled from the bone segments 28, 30. The medical professional then, if desired, uses the first and/or second plate adjustment mechanisms 78, 79 to adjust the distance 80 between the first and second bone segments 28, 30. For example, as shown in Figures 16-18B, the medical professional first loosens the fastener by at least one-half of a turn to unlock the bearing with respect to the frame portion. The medical professional may then use a bearing key 142 to rotate the first and/or second bearings 84, 86 with respect to the frame portion 68 of the orthopedic plate 14. The bearing key 142 shown in the figures includes a first key tooth 144 and a second key tooth 146 that correspond to and fit within the bearing key holes 148, 150, respectively. The bearing key holes 148, 150 are spaced apart and shaped so as to permit the medical professional to apply a torque force thereon and rotate the bearings 84, 86, thereby permitting dynamic compression of the bone segments 28, 30. For example, the bearing key holes 148, 150 shown in the figures are generally opposite each other on the bearings 84, 86. Additionally, the bearing key holes 148, 150 shown in the figures have a generally curved shape to promote a torque force on the bearings 84, 86.

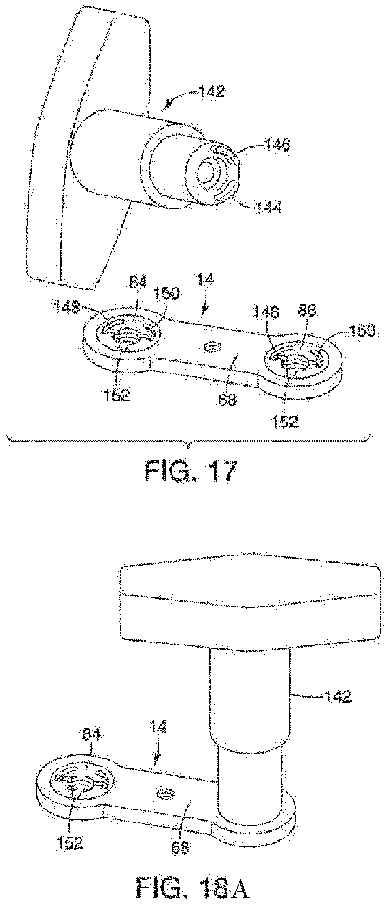

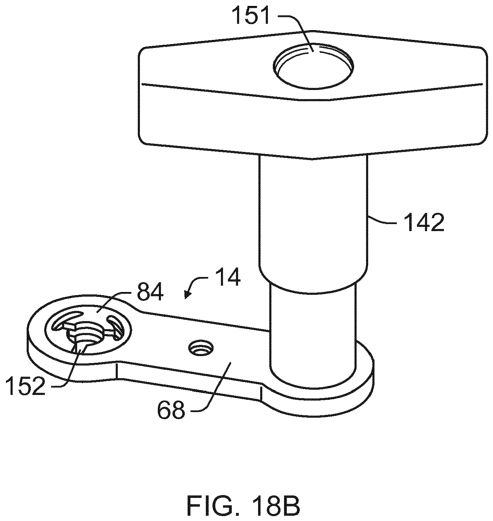

The desired distance 80 may vary depending on various circumstances, but it is typically 0.00 to 0.05 millimeters. After rotationally adjusting the bearings and obtaining a desired distance 80 and, if applicable, compression force, the medical professional tightens the fasteners so the fastener heads are flush with the bearings and the bearings are locked with respect to the frame portion. The fastener heads shown in the figures are conical, but they may be flat or any other shape. As best shown in Figures 17 and 18B, the bearing key 142 can define a fastener port 151 for inserting a tightening tool (not shown) through the bearing key 142 to engage the fastener heads and tighten the fastener. The fastener port 151 can be positioned to align with a fastener positioned to engage the first or second bearing when the bearing key 142 is coupled to the first or second bearing 84, 86. In this configuration, the bearing key 142 can remain coupled to the first or second bearing 84, 86 while the tightening tool is inserted through the bearing key 142.

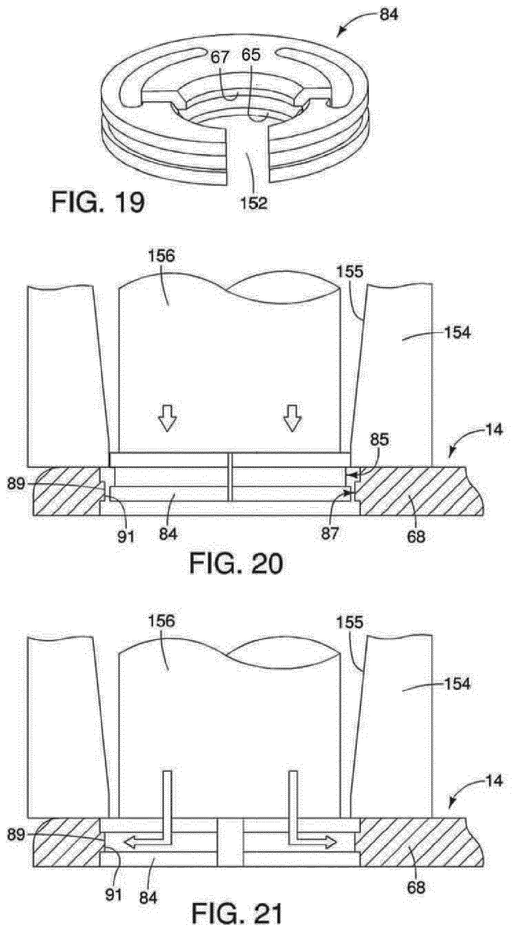

As shown in Figures 19-21, the bearing 84 may be coupled with the frame portion 68 of the orthopedic plate 14 via a spring connection. For example, the bearing has a notch 152 that allows the bearing to act like a C-shaped spring and compress and expand depending on the lateral force applied. The bearing 84 is inserted within a guide sleeve 154 that is coupled with or positioned flush with the frame portion 68 of the orthopedic plate 14. A punch mechanism 156 pushes down on the bearing 84 and forces it downward in the guide sleeve 154, until the bearing 84 is in a desired position with respect to the frame portion 68. For example, in the embodiment shown in the figures, the bearing 84 is in the desired location when an outer surface 85 of the bearing contacts an inner surface 87 of the frame portion 68. More specifically, in the embodiment shown in the figures, the bearing 84 is in the desired location when it snaps into place in the frame portion 68. The outer surface 85 of the bearing 84 includes a notch 89 that is configured to mate with a ring 91 in the inner surface 87 of the frame portion 68 to secure the bearing 84 with respect to the frame portion 68. The guide sleeve 154 shown in the figures includes an inner wall 155 that is tapered inwardly to increase the compression of the bearing 84 as it is forced downward by the punch mechanism 156.

In one alternative embodiment, as shown in Figure 23, an orthopedic plate 214 is provided having a bearing 268 with a first notch 352 in one portion of the bearing 268 and a second notch 356 diametrically opposed to the first notch 352 to alter the spring coefficient of the bearing 268 compared to the bearing shown in the prior figures. The bearing key holes 348, 350 are also larger than those shown in the prior figures and define portions of the outer surface of the bearing 268.

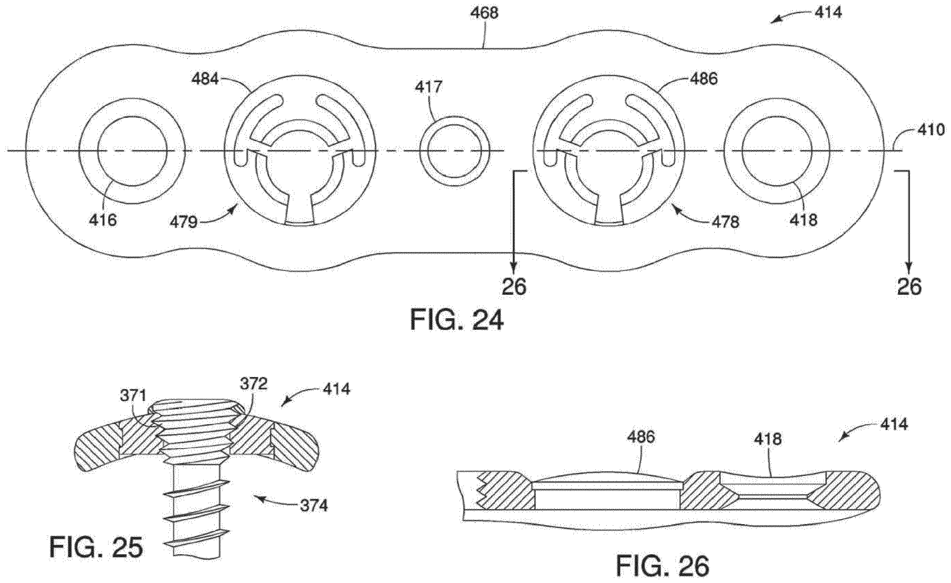

In another alternative embodiment, as shown in Figures 24-26, an orthopedic plate 414 is provided having a frame portion 468 and first and second plate adjustment mechanisms 478, 479 that are each configured to adjust a distance between first and second fasteners received within the plate adjustment mechanisms 478, 479. The plate adjustment mechanisms 478, 479 shown in the figure include first and second bearing 484, 486 that are each rotatable with respect to the frame portion 468. The frame portion 468 is generally curved along a longitudinal axis 410 so as to match the curvature of a bone. The orthopedic plate 414 also defines openings 412, 413, 414 for receiving fasteners and thereby further securing the orthopedic plate 414 to the bone. Alternatively, the opening 413 may be used as a connection point to a jig 12 or other device.

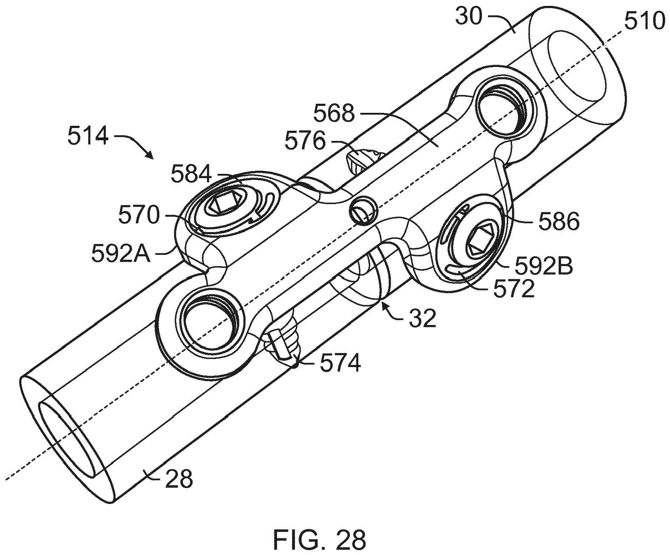

In one alternative embodiment, as shown in Figures 28-31B, an orthopedic plate 514 can be provided with a frame portion 568 having a longitudinal body defining a longitudinal axis 510. The longitudinal body can be positioned to extend across a fracture 32 between a first bone segment 28 and a second bone segment 30. The longitudinal body can comprise a first portion for interfacing with the first bone segment 28, wherein a first side arm 592A extends outward from the first portion of the longitudinal body. The longitudinal body can comprise a second portion for interfacing with the second bone segment 30, wherein a second side arm 592B extends outward from the second portion of the longitudinal body. In at least one embodiment, the longitudinal body can include at least one fixation opening 599 for receiving a fixation fastener to fixedly secure the longitudinal body to at least one of the first and second bone segments 28, 30 after the first and second bone segments 28, 30 are rejoined.

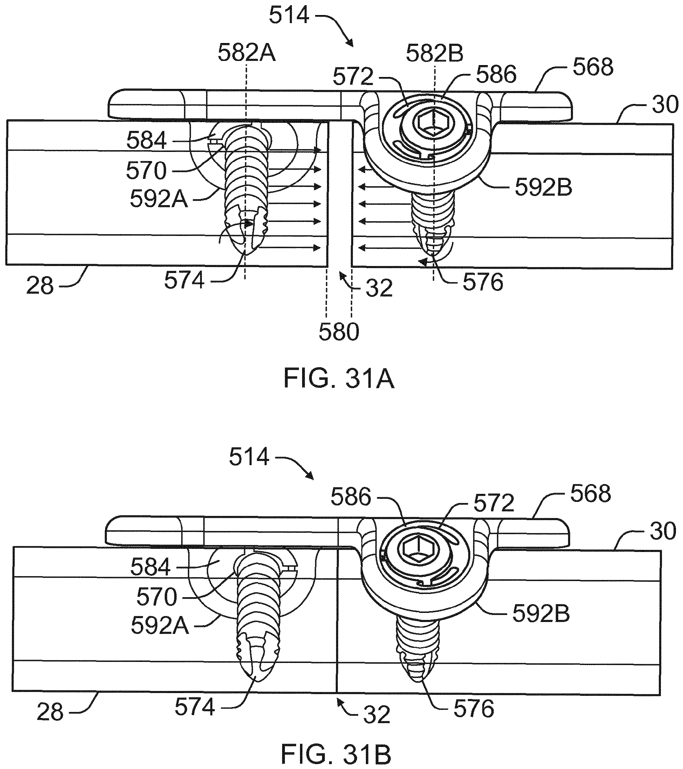

As best shown in Figures 28 and 31A-B, in an embodiment, the first side arm 592A can define an opening/port for rotatably receiving a first bearing 584, the first bearing 584 defining a first opening 570 for receiving a first fastener 574. The first fastener 574 can be advanced along a first axis 582A through the first opening 570 to engage the first bone segment 28 and operably secure the first bone segment 28 to the first bearing 584. Correspondingly, the second side arm 592B can define an opening/port for rotatably receiving a second bearing 586, the second bearing 586 defining a second opening 572 for receiving a second fastener 576. The second fastener 576 can be advanced along a second axis 582B through the second opening 572 to engage the second bone segment 30 and operably secure the second bone segment 30 to the second bearing 586. In this configuration, the longitudinal body operates to secure the first bone segment 28 and the second bone segment 30 together across the fracture 32 when the first fastener 574 and the second fastener 576 are secured to the respective bone segments 28, 30.

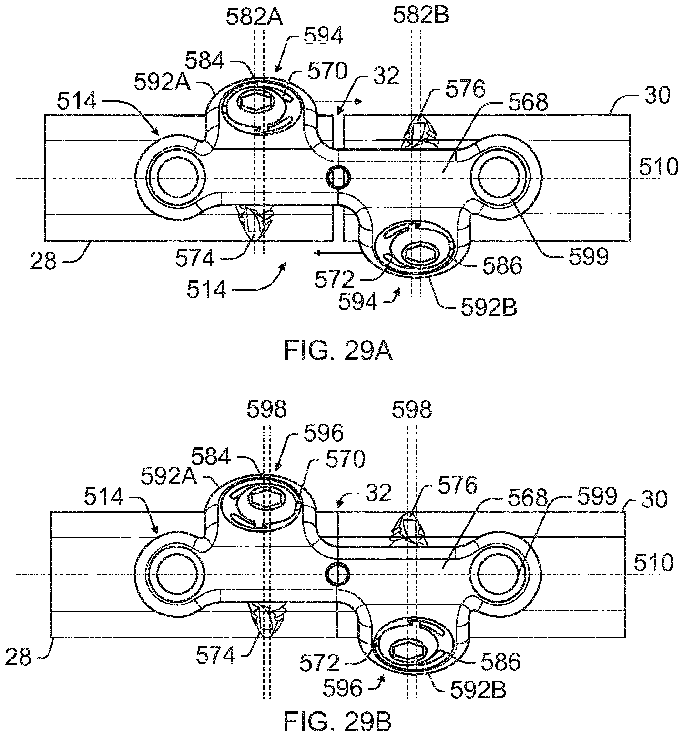

As best shown in Figures 29A, in an embodiment, the first bearing 584 can be rotated about a first rotational axis, wherein the first opening 570 is offset from the first rotational axis. The second bearing 586 can be rotated about a second rotational axis, wherein the second opening 572 is offset from the second rotational axis. In this configuration, the offset of the openings 570, 572 from the rotational axis moves the openings 570, 572 between a non-compressed position 594 and a compressed position 596. For example, the opening 570 for the first bearing 584 is oriented away from the second portion in the non-compressed position 594 and oriented toward the second portion in the compressed position 596. Correspondingly, the opening 572 for the second bearing 584 is oriented away from the first portion in the non-compressed position 594 and oriented toward the first portion in the compressed position 596.

As best shown in FIG. 29B, each of the first bearing 584 and the second bearing 586 can define a compression adjustment distance 598 corresponding to the distance between the non-compressed position 594 and the compressed position 596 along a compression axis parallel to the longitudinal axis 510 defined by the longitudinal body. In this configuration, the first bearing 584 and the second bearing 586 can be rotated between the compressed position and non-compressed position to change a distance 580 between the first fastener 574 and the second fastener 576. When the first fastener 574 and the second fastener 576 are engaged to the corresponding bone segments 28, 30, rotating the bearings 584, 586 can change the distance 580 between the first bone segment 28 and the second bone segment 30. The distance 580 is therefore adjustable by an amount equal to the compression adjustment distance 598 of the first bearing 584 plus the compression adjustment distance 598 of the second bearing 586. The orthopedic plate 514 can have a compression adjustment distance of approximately 1.5 millimeters, thereby allowing a medical professional to adjust the distance 580 of the orthopedic plate 514 by approximately 3.0 millimeters.

As best shown in Figures 29A-B, the first side arm 592A and the second side arm 592B can extend outward from the longitudinal body along an axis generally transverse to the longitudinal axis 510. In at least one embodiment, the first side arm 592A can extend outward from the longitudinal body on a first side of the longitudinal axis 510 and the second side arm 592B can extend outward from the longitudinal body on a second side of the longitudinal axis 510. In this configuration, the first side of the longitudinal body is positioned opposite to the second side of the longitudinal body. In this configuration, the first side arm 592A and the second side arm 592B can be oriented such that the first fastener 574 and the second fastener 576 extend into the first bone segment 28 and the second bone segment 30 from opposite directions. Rotation of the first bearing 584 (and the corresponding first fastener 574) can move the first bone segment 28 toward the second bone segment 30 to reduce the distance 580. Similarly, rotation of the second bearing 586 (and the corresponding second fastener 576) can move the second bone segment 30 toward the first bone segment 28 to reduce the distance 580. In an embodiment, the angle of the first side arm 592A relative to the angle of the second arm 592B can cause the first bone segment 28 to move into engagement with the second bone segment 30 along arcs in non-linear, intersecting arcs. This configuration can improve the alignment of the first bone segment 28 to the second bone segment 30. In an embodiment, the longitudinal body can provide a third point of contact that cooperates with the first fastener 574 and the second fastener 576 to further improve alignment of the first bone segment to the second bone segment 30. The opposing orientation of the first side arm 592A and the second side arm 592B can also apply compression forces on opposing sides of the fracture 32, which more evenly mates the first bone segment 28 and the second bone segment 30 across the fracture 32. In an embodiment, the planar longitudinal body can extend across the fracture 32 between the first bone segment 28 and the second bone segment 30.

As best shown in Figures 31A-B, the first axis 582A can be parallel to the end of the first bone segment 28 at the fracture 32 such that the first fastener 574 is oriented parallel to the end of the first bone segment 28. Similarly, the second axis 582B can be parallel to the end of the second bone segment 30 at the fracture 32 such that the second fastener 576 is oriented parallel to the end of the second bone segment 30. In this configuration, rotation of the first and second bearings 584, 586 applies compression forces inward toward the fracture 32 along the length of the first and second fastener 574, 576 as best shown in Figure 31A.

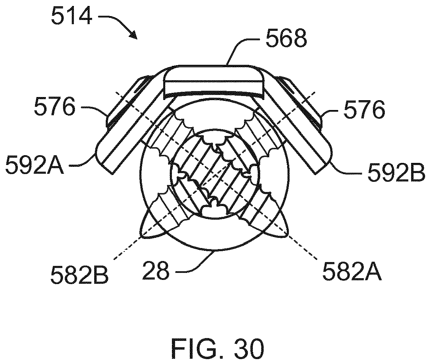

As best shown in Figures 30 and 31A-B, the longitudinal body can generally define a plane when positioned across the fracture 32 between the first bone segment 28 and the second bone segment 30. The first side arm 592A and/or the second side arm 592B can extend from the longitudinal body along axes transverse to the plane defined by the longitudinal body. In this configuration, the first side arm 592A and/or the second side arm 592B can cooperate with the longitudinal body to correspond to the shape and contours of the first and second bone segments 28, 30. In this configuration, the first side arm 592A and the second side arm 592B can be oriented at the same deflection angle or angled at different offset angles. In at least one embodiment, the first side arm 592A, the second side arm 592B, or both can be oriented to be parallel to the plane defined by the longitudinal body to correspond to the curvature of the underlying bone.

As best shown in Figures 30 and 31A-B, the deflection of the first side arm 592A and/or the second side arm 592B relative to the longitudinal axis 510 alters the first and second axis 582A, 582B along which the corresponding first or second fastener 574, 576 are inserted. In an embodiment, the first fastener 574 and the second fastener 576 are oriented along transverse axes when viewed along the longitudinal axis 510. In the offset orientation, the compression forces applied to the first and second bone segments 28, 30 at the fracture 32 from the first fastener 574 and the second fastener 576 are offset to apply compression forces over a greater surface area of a face of the fracture 32. As defined in the present disclosure, the face of the fracture 32 is the adjoining surfaces of the first and second bone segments 28, 30 that are rejoined. As best shown in Figure 30, in an example, the first and second fasteners 574, 576 can extend through most of or the entirety of the corresponding first and second bone segments 28, 30. In this configuration, the first side arm 592A and the second side arm 592B are oriented such that the first and second fasteners 574, 576 are along mirroring axes when viewed along the longitudinal axis 510 to evenly distribute the compression forces across the face of the fracture 32.

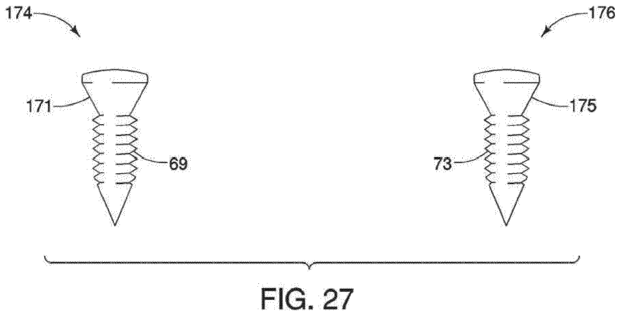

As shown in Figure 27, in an alternative design, fasteners 174, 176 include a locking head 171, 175 configured to be received by the locking ridge 67 of the bearings. The locking head 171, 175 is tapered so as to increase the radially expansion of the diameter of the bearings as the locking head 171, 175 is driven downward with respect to the orthopedic plate. As with the locking threads 71, 75, the locking heads 171, 175 are configured to mate with the locking ridge 67 when the fasteners 17 4, 176 are substantially or completely screwed down into the bone segments 28, 30 so as to prevent the bearings from rotating with respect to the plate portion 68. More specifically, the diameter of the locking threads 171, 175 is sized so as to cause the bearings 84, 86 to expand and form a friction engagement with the plate portion 68. The bearings 84, 86 are able to rotate with respect to the plate portion 68 except when the locking threads 171, 175 cause the bearings 84, 86 to expand and form a friction engagement with the plate portion 68.

Figure 25 shows another alternative design for a fastener 374, where a locking head 371 is tapered so as to increase the radially expansion of the diameter of the bearings as the locking head 371 is driven downward with respect to the orthopedic plate 414. Additionally, the locking head 371 includes threads 372 configured to mate with threads in the orthopedic plate 414.

### Various Notes & Examples

Example 1 is an orthopedic plate assembly for joining a first bone segment and a second bone segment, comprising: a frame portion comprising: a longitudinal body comprising a first portion for interfacing with the first bone segment and a second portion for interfacing with the second bone segment, and a first side arm extending outwards from the first portion of the longitudinal body; a first fastener; a first bearing rotatably coupled with the side arm and defining an opening configured to receive the first fastener, wherein the first fastener is advanceable in the opening in a first direction to a set position in which the first fastener engages the first bone segment; wherein the first bearing is rotatable about a first rotational axis, wherein the opening is offset from the first rotational axis.
In Example 2, the subject matter of Example 1 optionally includes wherein the first bearing is rotatable about the first rotational axis between a non-compressed position where the opening is positioned distal to the second portion and a compressed position where the opening is positioned proximate to the second portion.
In Example 3, the subject matter of Example 2 optionally includes wherein the frame portion further comprises: a second side arm extending outwards from the second portion of the longitudinal body.
In Example 4, the subject matter of Example 3 optionally includes a second fastener; a second bearing rotatably coupled with the second side arm and defining an opening configured to receive the second fastener, wherein the second fastener is advanceable in the opening in a second direction to a set position in which the second fastener engages the second bone segment; wherein the second bearing is rotatable about a second rotational axis, wherein the opening is offset from the second rotational axis.
In Example 5, the subject matter of Example 4 optionally includes wherein the second bearing is rotatable about the second rotational axis between a non-compressed position where the opening is positioned distal to the first portion and a compressed position where the opening is positioned proximate to the first portion.
In Example 6, the subject matter of Example 5 optionally includes wherein the first fastener is advanceable into the set position when the first bearing is in the non-compressed position such that rotating the first bearing into the compressed position draws the first bone segment toward the second portion of the longitudinal body; wherein the second fastener is advanceable into the set position when the second bearing is in the non-compressed position such that rotating the second bearing into the compressed position draws the second bone segment toward the first portion of the longitudinal body and into engagement with first bone segment.
In Example 7, the subject matter of Example 6 optionally includes wherein the first fastener is advanceable in the first opening to a lock position when the first bearing is rotated into the compressed position, wherein an expansion portion of the first fastener is configured to engage the first bearing in the lock position to cause the first bearing to expand radially and become non-rotatably locked in the first side arm.
In Example 8, the subject matter of any one or more of Examples 6-7 optionally include wherein the second fastener is advanceable in the second opening to a lock position when the second bearing is rotated into the compressed position, wherein an expansion portion of the second fastener is configured to engage the second bearing in the lock position to cause the second bearing to expand radially and become non-rotatably locked in the second side arm.
In Example 9, the subject matter of any one or more of Examples 3-8 optionally include wherein the first side arm extends from the first portion on a first side of the longitudinal body and the second side arm extends from the second portion on a second side of the longitudinal body, wherein the first side is opposite to the second side.
In Example 10, the subject matter of any one or more of Examples 3-9 optionally include wherein the longitudinal body comprises a planer shape defining a plane.
In Example 11, the subject matter of Example 10 optionally includes wherein at least one of the first side arm and the second side arm extends from the longitudinal body transversely from the plane defined by the longitudinal body.
In Example 12, the subject matter of any one or more of Examples 1-11 optionally include wherein at least one of the first portion and the second portion defines a set opening for receiving a set fastener for fixing the longitudinal body to at least one of the first bone segment and the second bone segment.
Example 13 is a method of connecting a first bone segment to a second bone segment with an orthopedic plate assembly, comprising: positioning a longitudinal body of a frame portion of the orthopedic plate assembly such that a first portion of the longitudinal body is adjacent to the first bone segment and a second portion of the longitudinal body is adjacent to the second bone segment; coupling a first bearing to a first side arm extending outwards from the first portion of the frame portion, the bearing comprising an opening that defines a first direction of travel; advancing a first fastener in the first direction to a set position in which the first fastener engages the first bone segment; and rotating the first bearing about a first rotational axis, wherein the opening is offset from the first rotational axis.
In Example 14, the subject matter of Example 13 optionally includes wherein the first bearing is rotatable about the first rotational axis between a non-compressed position where the opening is positioned distal to the second portion and a compressed position where the opening is positioned proximate to the second portion.
In Example 15, the subject matter of Example 14 optionally includes coupling a second bearing to a second side arm extending outwards from the second portion of the frame portion, the bearing comprising an opening that defines a second direction of travel; advancing a second fastener in the second direction to a set position in which the second fastener engages the first bone segment; and rotating the second bearing about a second rotational axis, wherein the opening is offset from the second rotational axis.
In Example 16, the subject matter of Example 15 optionally includes wherein the second bearing is rotatable about the second rotational axis between an non-compressed position where the opening is positioned distal to the first portion and a compressed position where the opening is positioned proximate to the first portion.
In Example 17, the subject matter of Example 16 optionally includes wherein rotating the first bearing to the compressed position and the second bearing to the compressed position draws the first bone segment and the second bone segment into engagement.
In Example 18, the subject matter of Example 17 optionally includes advancing the first fastener in the first direction to a lock position where an expansion portion of the first fastener engages the first bearing causing the first bearing to expand radially and become non-rotatably locked in the first side arm.
In Example 19, the subject matter of any one or more of Examples 17-18 optionally include advancing the second fastener in the second direction to a lock position where an expansion portion of the second fastener engages the second bearing causing the second bearing to expand radially and become non-rotatably locked in the second side arm.
In Example 20, the subject matter of any one or more of Examples 17-19 optionally include wherein the first bone segment and the second bone segment are moved into engagement along non-linear paths.
In Example 21, the subject matter of any one or more of Examples 15-20 optionally include wherein the longitudinal body comprises a planer shape defining a plane.
In Example 22, the subject matter of any one or more of Examples 19-21 optionally include wherein at least one of the first side arm and the second side arm extends from the longitudinal body transversely from the plane defined by the longitudinal body.
In Example 23, the subject matter of any one or more of Examples 13-22 optionally include advancing a set fastener through a set opening in at least one of the first portion and the second portion of the longitudinal body to engage at least one of the first bone segment and the second bone segment.

Each of these non-limiting examples can stand on its own, or can be combined in any permutation or combination with any one or more of the other examples.

The above detailed description includes references to the accompanying drawings, which form a part of the detailed description. The drawings show, by way of illustration, specific embodiments in which the present subject matter can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the present subject matter should be determined with reference to the appended claims, along with the full scope of equivalents to which such claims are entitled.

## Claims

1. An orthopedic plate assembly for joining a first bone segment and a second bone segment, comprising:
a frame portion comprising:
a longitudinal body comprising a first portion for interfacing with the first bone segment and a second portion for interfacing with the second bone segment, and
a first side arm extending outwards from the first portion of the longitudinal body;
a first fastener;
a first bearing rotatably coupled with the side arm and defining an opening configured to receive the first fastener, wherein the first fastener is advanceable in the opening in a first direction to a set position in which the first fastener engages the first bone segment;
wherein the first bearing is rotatable about a first rotational axis, wherein the opening is offset from the first rotational axis.

2. The orthopedic plate assembly of claim 1, wherein the first bearing is rotatable about the first rotational axis between a non-compressed position where the opening is positioned distal to the second portion and a compressed position where the opening is positioned proximate to the second portion.

3. The orthopedic plate assembly of claim 2, wherein the frame portion further comprises:
a second side arm extending outwards from the second portion of the longitudinal body.

4. The orthopedic plate assembly of claim 3, further comprising:
a second fastener;
a second bearing rotatably coupled with the second side arm and defining an opening configured to receive the second fastener, wherein the second fastener is advanceable in the opening in a second direction to a set position in which the second fastener engages the second bone segment;
wherein the second bearing is rotatable about a second rotational axis, wherein the opening is offset from the second rotational axis.

5. The orthopedic plate assembly of claim 4, wherein the second bearing is rotatable about the second rotational axis between a non-compressed position where the opening is positioned distal to the first portion and a compressed position where the opening is positioned proximate to the first portion.

6. The orthopedic plate assembly of claim 5, wherein the first fastener is advanceable into the set position when the first bearing is in the non-compressed position such that rotating the first bearing into the compressed position draws the first bone segment toward the second portion of the longitudinal body;
wherein the second fastener is advanceable into the set position when the second bearing is in the non-compressed position such that rotating the second bearing into the compressed position draws the second bone segment toward the first portion of the longitudinal body and into engagement with first bone segment.

7. The orthopedic plate assembly of claim 6, wherein the first fastener is advanceable in the first opening to a lock position when the first bearing is rotated into the compressed position, wherein an expansion portion of the first fastener is configured to engage the first bearing in the lock position to cause the first bearing to expand radially and become non-rotatably locked in the first side arm.

8. The orthopedic plate assembly of claim 6, wherein the second fastener is advanceable in the second opening to a lock position when the second bearing is rotated into the compressed position, wherein an expansion portion of the second fastener is configured to engage the second bearing in the lock position to cause the second bearing to expand radially and become non-rotatably locked in the second side arm.

9. The orthopedic plate assembly of claim 3, wherein the first side arm extends from the first portion on a first side of the longitudinal body and the second side arm extends from the second portion on a second side of the longitudinal body,
wherein the first side is opposite to the second side.

10. The orthopedic plate assembly of claim 3, wherein the longitudinal body comprises a planer shape defining a plane.

11. The orthopedic plate assembly of claim 10, wherein at least one of the first side arm and the second side arm extends from the longitudinal body transversely from the plane defined by the longitudinal body.

12. The orthopedic plate assembly of claim 1, wherein at least one of the first portion and the second portion defines a set opening for receiving a set fastener for fixing the longitudinal body to at least one of the first bone segment and the second bone segment.
